# FASCICULE DE BREVET EUROPEEN

(11) **EP 3 314 263 B1**
(45) Date de publication et mention de la délivrance du brevet: **15.12.2021**
(21) Numéro de dépôt: 16744862.0
(22) Date de dépôt: 24.06.2016
(51) Int. Cl.: G01N 33/543, G01N 33/558

(54) **DISPOSITIF A FLUX LATERAL AVEC PRELEVEMENT**
LATERAL-FLOW-VORRICHTUNG MIT PROBENNAHME
LATERAL FLOW DEVICE COMPRISING SAMPLING

(30) Priorité: 25.06.2015 FR 1555890
(43) Date de publication de la demande: 02.05.2018
(73) Titulaire: Commissariat à l'Énergie Atomique et aux Énergies Alternatives, 75015 Paris (FR); Societe des Systemes Biologiques, 38140 Apprieu (FR); Université Grenoble Alpes, 38400 Saint Martin d'Hères (FR); Centre Hospitalier Universitaire Grenoble Alpes, 38043 Grenoble Cedex 9 (FR)
(72) Inventeur: MOMBRUN, Adrien, 38000 Grenoble (FR); BERGER, François, 38240 Meylan (FR); BOUAMRANI, Mohamed-Ali, 38000 Grenoble (FR); BRANDAM, Gary, 69540 Irigny (FR); DEFFAUD, Clarence, 38500 Voiron (FR); ROUX, Julien, 38500 Voiron (FR)
(74) Mandataire: Hautier, Nicolas
(86) Numéro de dépôt international: PCT/IB2016/053766
(87) Numéro de publication internationale: WO 2016/207842

(56) Documents cités:
- WO-A1-96/10179
- WO-A1-97/34150
- WO-A1-2009/003177
- WO-A1-2013/140089
- WO-A2-2004/067162
- WO-A2-2007/023372
- BE-A3- 1 011 487
- FR-A1- 2 853 077
- FR-A1- 2 881 828
- FR-A1- 2 967 497
- FR-A3- 2 614 423

## Description

L'invention se situe dans le domaine des dispositifs à usage unique de diagnostic rapide et qualitatif.

Les dispositifs classiques à flux latéral permettent une détection colorimétrique d'analytes présents dans un fluide. Ces dispositifs mettent en œuvre un matériau absorbant, à travers lequel migre le fluide à analyser. Ils sont particulièrement adaptés à une collecte *in situ* d'un liquide corporel, à une filtration de ce liquide (par exemple dans le cas du sang, le retrait des érythrocytes), ainsi qu'à une analyse qualitative du fluide, c'est-à-dire une première information sur la présence ou l'absence d'une certaine quantité d'un analyte donné dans le liquide. De l'art antérieur, on connaît notamment les documents WO 96/10179 A1 et FR 2 853 077 A1 qui mettent en œuvre des dispositifs en phase solide pour détecter un analyte d'un échantillon liquide.

Du document US 2002/0036170 on connaît un dispositif à flux latéral conçu pour séparer les globules rouges du plasma sanguin. Le dispositif comporte une languette sur laquelle est déposée une membrane chromatographique à flux latéral ; il comporte, à une première extrémité de la membrane, une zone d'application d'échantillon, suivie d'une zone de capture comportant un agent de liaison avec des érythrocytes, puis une zone de capture à une seconde extrémité. L'objectif est de capturer une ou plusieurs protéine(s) d'intérêt dans la zone de capture en vue d'une analyse soit visuelle (qualitative), soit par prélèvement d'une portion de membrane dans la zone de capture (quantitative).

Le document US 2012/0308444 décrit un dispositif à flux latéral conçu pour identifier simultanément la présence de deux protéines dans un échantillon de liquide. Le dispositif comporte une languette sur laquelle est déposée une membrane chromatographique à flux latéral en nitrocellulose ; il comporte sur la membrane successivement une zone d'application d'échantillon, une première puis une seconde zone de conjugaison, une première ligne de lecture pour révéler la présence de la première protéine, une deuxième ligne de test pour révéler la présence de la deuxième protéine et une ligne de contrôle pour révéler que le liquide a franchi les deux lignes de test.

Le document US 2006/0019404 décrit un dispositif à flux latéral permettant une mesure au moins partiellement quantitative d'un analyte d'intérêt par l'utilisation de différentes zones de test, de sensibilités différentes. Une mesure quantitative de la concentration en hCG est obtenue par mesure de la réflectance de la couleur bleue obtenue dans la zone de capture.

De tels dispositifs ne sont pas compatibles avec les méthodes d'analyses quantitatives précises, par exemple une spectrométrie de masse.

Aussi, on cherche à disposer d'un dispositif qui, tout en permettant une mesure qualitative (par exemple un changement de couleur lorsqu'un analyte est présent dans le liquide), permette d'y associer une mesure quantitative (un dosage), en particulier lorsque la mesure qualitative conduit à une suspicion de présence d'un analyte particulier.

De plus, on cherche à disposer d'un dispositif propice à une utilisation *in situ,* au lit du malade si nécessaire et sans nécessiter d'équipement particulier sur son lieu d'utilisation, et qui permette malgré cela de réaliser, à partir du même prélèvement, une analyse moléculaire précise et quantitative, éventuellement différée dans le temps par rapport au moment de la collecte. Ceci implique que l'échantillon ait été stabilisé.

Pour résoudre ces difficultés, l'invention propose un dispositif à flux latéral pour le prélèvement d'au moins un analyte d'intérêt dans un échantillon liquide, comportant :
- Une zone de réception du liquide, comportant un premier matériau absorbant apte à recueillir le liquide,
- Au moins une zone de migration du liquide, s'étendant entre une première extrémité et une deuxième extrémité, chaque zone de migration comportant un matériau drainant en continuité fluidique avec le premier matériau absorbant, le matériau drainant étant apte à collecter le liquide absorbé par la zone de réception et à drainer le liquide de sa première extrémité vers sa deuxième extrémité,
- Au moins une zone réservoir, comportant un deuxième matériau absorbant, en contact avec le matériau drainant au niveau de la deuxième extrémité de chaque zone de migration, le deuxième matériau absorbant étant apte à absorber le liquide drainé par le matériau drainant,
- Des moyens de détection entre la zone de réception et la zone réservoir, permettant de déterminer la présence ou l'absence du ou des analytes d'intérêt dans l'échantillon,

dans lequel au moins l'une des zones de migration comporte également au moins une zone de capture, le dispositif comprenant en outre un élément de capture appliqué sur chaque zone de capture, contre le matériau drainant entre sa première et sa deuxième extrémité, chaque élément de capture comportant un matériau nanoporeux ou mésoporeux en silicium et étant apte à capturer un analyte d'intérêt présent dans le liquide et en dans lequel chaque élément de capture est apte à être séparé du matériau drainant. Le terme « analyte d'intérêt » désigne un analyte dont on souhaite détecter la présence dans le liquide. Il peut s'agir d'une protéine, d'une molécule organique, d'une drogue, d'une toxine, d'un acide nucléique, d'un sucre, d'un lipide, de traces de micro-organismes ou de parasites, *etc.*

Par « échantillon liquide » on entend tout type d'échantillon liquide provenant d'un environnement à analyser, et en particulier tout type d'échantillon de liquide corporel tel que le sang, le plasma, le sérum, l'urine, le liquide ascitique, le liquide pleural, le liquide péricardique, le liquide synovial ou un lavage broncho-alvéolaire. Des liquides environnementaux, tels que des effluents, peuvent également être analysés en utilisant un dispositif selon la présente invention.

Par « en continuité fluidique », on entend que le matériau absorbant et le matériau drainant sont disposés de telle sorte que le liquide déposé sur le premier matériau absorbant peut progresser par capillarité vers et dans le matériau drainant. Par exemple ces matériaux sont directement en contact, ou bien ils sont reliés par un autre matériau à travers lequel le liquide peut également progresser par capillarité.

On tire profit des capacités de filtration du premier matériau absorbant et du matériau drainant de façon à ce que les plus grosses particules ou cellules présentes dans le liquide en soient soustraites au moment où il atteint la zone de capture. Ainsi, le rendement de collecte d'analytes d'intérêts, et notamment des petites molécules, est optimisé, et compatible avec une lecture directe en spectrométrie de masse. L'expérience montre que la position de la zone de capture, et donc du matériau de capture, entre la zone de réception et la zone réservoir a une influence sur la nature, notamment la taille, des analytes collectés et enrichis sur le matériau de capture (ceci est illustré par l'exemple 1 ci-après).

La demande n'exclut nullement que le dispositif comporte plusieurs zones de capture, notamment deux zones de capture, en particulier lorsqu'il comporte deux zones de migration.

Dans cette configuration, l'élément de capture est appliqué sur la zone de capture du matériau drainant.

Le Silicium nanoporeux ou mésoporeux a l'avantage de conserver les analytes d'intérêt piégés pendant plusieurs jours sans qu'ils se dégradent (ceci est illustré par l'exemple 2 ci-après).

Avantageusement, les moyens de détection peuvent comporter :
- Au moins un matériau de conjugaison disposé au contact du matériau drainant, le matériau de conjugaison comportant des particules de conjugaison aptes à se lier au(x) analyte(s) d'intérêt,
- un matériau apte à immobiliser les particules de conjugaison liées à un analyte d'intérêt et à les révéler, ledit matériau imprégnant une zone de détection du matériau drainant. Un tel matériau de conjugaison (ou *conjugate pad*) peut être disposé à cheval sur deux segments séparés du matériau drainant pour garantir qu'il soit traversé par le liquide.

Les particules de conjugaison peuvent notamment être des microbilles fonctionnalisées de façon à se lier au(x) analyte(s) d'intérêt.

La zone de détection est une zone du matériau drainant imprégnée d'un matériau apte à immobiliser les particules de conjugaison liées à un analyte d'intérêt, située en aval du matériau de conjugaison dans le sens de drainage du liquide. Les particules de conjugaison sont généralement colorées ou fluorescentes, de façon à ce que lorsque le liquide comporte une certaine quantité d'analytes d'intérêt, la zone de détection capte lesdits analytes greffés sur les microbilles et change ainsi d'aspect. On peut alors avoir une lecture colorimétrique, qualitative, représentative de la quantité d'analyte d'intérêt dans le liquide prélevé. L'excès de particules de conjugaison est drainé vers la zone réservoir.

Dans un second mode de réalisation, le dispositif peut comporter une première et une seconde zones de migration comportant respectivement un premier et un second matériau drainant, la zone de réception étant située entre lesdites deux zones de migration.

La zone de réception est en contact fluidique avec la première extrémité de chaque zone de migration, chaque zone réservoir étant en contact avec la seconde extrémité d'une zone de migration respective. Lorsque l'échantillon est déposé sur la zone de réception, le liquide diffuse de part et d'autre de cette zone. Une première partie du liquide est utilisée pour une détection qualitative, par exemple colorimétrique, et une deuxième partie du liquide est absorbée par le matériau de capture, lequel peut être analysé en cas de détection positive.

Les premier et second matériaux drainant peuvent être identiques ou différents.

Avantageusement, le premier et/ou le second des matériaux absorbants peuvent être réalisés dans un matériau fibreux.

Avantageusement, la zone de réception peut comporter, outre le premier matériau absorbant, un matériau de transfert.

Ce matériau de transfert assure alors la continuité fluidique entre le premier matériau absorbant et un matériau drainant.

Avantageusement, au moins l'un des matériaux drainant peut comporter un matériau nanoporeux ou mésoporeux. Le fait que ce matériau ne soit pas fibreux permet que le liquide progresse selon un front sensiblement rectiligne.

Avantageusement, la zone de capture peut être située en amont de la zone de conjugaison dans le sens du drainage du liquide dans le matériau drainant. On s'assure ainsi que le matériau de capture ne sera pas pollué par des particules de conjugaison.

Avantageusement, au moins l'un des matériaux de conjugaison peut comporter des particules de conjugaison aptes à se lier à un analyte que l'on est certain de trouver dans le liquide.

De cette façon, la détection de ces particules de conjugaison dans la zone de détection, de préférence située en aval de la zone de capture, permet de s'assurer que le liquide a été exposé à l'élément de capture.

Avantageusement, le premier matériau absorbant, chaque matériau drainant et le second matériau absorbant sont disposés sur un matériau support.

Le matériau support est réalisé de préférence dans un matériau non absorbant, de façon à canaliser la migration du fluide.

Avantageusement, le dispositif peut comporter en outre un matériau de couverture apte à recouvrir au moins une partie de la zone de réception, la zone de migration et/ou la zone réservoir.

La fonction de ce matériau de couverture est d'isoler le dispositif de l'environnement, de façon à prévenir une contamination intempestive. Dans ce cas, un élément de capture peut être maintenu entre le matériau de couverture et le matériau de drainage.

Ceci permet de détacher l'élément de capture pour l'envoyer dans un laboratoire en vue d'une analyse quantitative de l'analyte recherché.

L'élément de capture et le premier matériau absorbant peuvent être disposés sur la même face ou sur deux faces opposées du matériau drainant.

Le fait de combiner, sur un même dispositif, une zone de détection colorimétrique et un élément de capture permet de disposer d'une première analyse qualitative, indiquant une possibilité de présence d'un analyte d'intérêt. Il est alors possible de détacher l'élément de capture et de l'analyser à l'aide d'une méthode quantitative de type spectrométrie de masse, éventuellement dans un temps différé, afin de disposer d'un dosage précis de l'analyte d'intérêt ainsi, éventuellement, que d'un dosage d'autres analytes.

Avantageusement, l'élément de capture peut être maintenu contre le matériau drainant.

Par « maintenu contre le matériau drainant » on entend que l'élément de capture n'est pas séparable du matériau drainant sans au moins partiellement détruire le dispositif. C'est par exemple le cas quand l'élément de capture est intercalé entre le matériau drainant et le matériau support, ou entre le matériau drainant et le matériau de couverture. Dans ce cas, l'élément de capture n'est pas nécessairement détaché du dispositif pour être envoyé dans un centre d'analyse possédant des moyens d'analyse quantitative ; le dispositif peut en effet être envoyé entier.

L'invention concerne également un système de prélèvement d'analytes d'intérêt dans un liquide, caractérisé en ce qu'il comporte une enceinte renfermant un dispositif à flux latéral selon l'invention, comportant une seule zone de migration, l'enceinte comportant :
- une première ouverture disposée en vis-à-vis de la zone de réception du dispositif,
- une première fenêtre disposée en vis-à-vis de la zone de détection afin de permettre de lire un signal de détection. L'invention concerne également
   un système de prélèvement d'analytes d'intérêt dans un liquide, caractérisé en ce qu'il comporte une enceinte renfermant un dispositif à flux latéral selon l'invention, comportant deux zones de migration, l'enceinte comportant :
   - une première ouverture disposée en vis-à-vis de la zone de réception du dispositif,
   - une première fenêtre disposée en vis-à-vis de la première zone de détection afin de permettre de lire un premier signal de détection,
   - une seconde fenêtre disposée en vis-à-vis de la seconde zone de détection afin de lire un second signal de détection.

De cette façon l'opérateur ne touche plus le dispositif dans sa manipulation, ce qui réduit le risque de contaminations qui seraient dues à cette manipulation.

Avantageusement, l'enceinte peut comporter en outre une deuxième ouverture disposée en vis-à-vis d'une zone de capture du dispositif, de façon à pouvoir déposer un élément de capture sur la zone de capture et/ou l'en retirer.

L'élément de capture est donc déposé sur le matériau drainant, au niveau de la zone de capture, au travers de la deuxième ouverture.

L'enceinte peut en outre comporter au moins un indicateur de position en vue de guider l'utilisateur dans la dépose de l'élément de capture sur le matériau drainant, par exemple sous la forme de graduations, ces indicateurs étant utilisés comme repères pour le positionnement du matériau de capture. Le matériau de capture sera d'autant plus éloigné de la zone de réception que l'on souhaite collecter des analytes de faible taille.

La première fenêtre et la deuxième ouverture peuvent être confondues en une ouverture unique.

Selon une première alternative, le dispositif peut comprendre en outre au moins un élément de capture maintenu sur la zone de capture, chaque élément de capture étant apte à capturer un analyte d'intérêt présent dans le liquide.

Dans ce cas, l'élément de capture ne peut pas être facilement désolidarisé du matériau drainant et le système entier, enceinte et dispositif, est préférentiellement envoyé au centre d'analyse.

Selon une seconde alternative, l'élément de capture peut être fixé à un support détachable de l'enceinte.

Dans ce cas, le support détachable de l'enceinte peut être l'un des éléments suivants :
- Une plaquette apte à se fixer au moins temporairement sur les bords de la deuxième ouverture,
- Une partie sécable de l'enceinte,
- Un bouchon apte à se fixer à la fois sur une capsule et sur la deuxième ouverture. Dans ce cas, seul le support détachable est envoyé au centre d'analyse.

La présente invention porte également sur un procédé de prélèvement d'au moins un analyte d'intérêt dans un échantillon à l'aide d'un dispositif ou d'un système comme décrits plus haut, comportant les étapes suivantes :
- déposer l'échantillon sur la zone de réception d'un dispositif à flux latéral, et
- attendre que le liquide ait migré jusqu'à la zone réservoir,
dans lequel, en cas de présence de l'analyte d'intérêt dans l'échantillon, avérée par les moyens de détection, ledit analyte est prélevé dans l'élément de capture.

Lorsque ce procédé de prélèvement est effectué à l'aide d'un dispositif qui prévoit la séparation de l'élément de capture, seul ou attaché à un support tel que, par exemple, le bouchon décrit plus haut, une étape additionnelle est avantageusement ajoutée, consistant, à l'issue de l'étape de migration du liquide, à séparer l'élément de capture du reste du dispositif . L'élément de capture peut être ensuite conservé pendant une durée de plusieurs semaines si nécessaire et/ou envoyé dans un laboratoire distant pour effectuer un dosage quantitatif de son contenu et/ou introduit dans un appareil de dosage tel qu'un appareil de spectrométrie de masse.

Un procédé de dosage d'au moins un analyte d'intérêt dans un échantillon de liquide, comportant une étape de prélèvement dudit/desdits analyte(s) par un procédé de prélèvement tel que décrit ci-dessus, suivie d'une étape de dosage dudit/desdits analyte(s) présent(s) dans l'élément de capture, est également partie intégrante de l'invention.

Un procédé de dosage particulier conforme à l'invention, à l'aide d'un système tel que décrit ci-dessus, comporte les étapes suivantes :
- déposer ledit échantillon sur la première ouverture de l'enceinte,
- attendre le temps nécessaire à la migration du liquide jusqu'à la zone de détection,
- lire un résultat établissant que l'échantillon de liquide a migré au-delà de la zone de capture et, si possible, que l'analyte d'intérêt est bien présent dans l'échantillon, et
- procéder au dosage d'au moins un analyte d'intérêt à partir de la quantité d'analyte capturée par l'élément de capture.

Selon un autre procédé de dosage conforme à l'invention, le procédé est effectué à l'aide d'un système dans lequel l'élément de capture est fixé à un support détachable de l'enceinte tel qu'une plaquette ou un bouchon ; le procédé comporte alors les étapes suivantes :
- le cas échéant (si le système est fourni avec l'élément de capture initialement séparé du reste), appliquer l'élément de capture sur la zone de capture du matériau drainant, à l'aide du support détachable,
- déposer ledit échantillon de liquide sur la première ouverture de l'enceinte,
- attendre le temps nécessaire à la migration du liquide jusqu'à la zone de détection,
- lire un résultat établissant que l'échantillon de liquide a migré au-delà de la zone de capture,
- détacher le support détachable de l'enceinte, et
- procéder au dosage d'au moins un analyte d'intérêt à partir de la quantité d'analyte capturée par l'élément de capture.

Selon une variante du procédé ci-dessus, dans laquelle le support détachable est une partie sécable de l'enceinte, le procédé comporte les étapes suivantes :
- déposer ledit échantillon de liquide sur la première ouverture de l'enceinte,
- attendre le temps nécessaire à la migration du liquide jusqu'à la zone de détection,
- lire un résultat établissant que l'échantillon de liquide a migré au-delà de la zone de capture,
- détacher la partie sécable de l'enceinte,
- procéder au dosage d'au moins un analyte d'intérêt à partir de la quantité d'analyte capturée par l'élément de capture.

Des modes de réalisation et des variantes seront décrits ci-après, à titre d'exemples non limitatifs, avec référence aux dessins annexés dans lesquels :
- Les figures 1A et 1B représentent en élévation deux modes de réalisation d'un dispositif,
- Les figures 2 et 3 représentent en perspective deux modes de réalisation d'un dispositif dans lequel un élément de capture est indissociable du dispositif,
- La figure 4 représente en perspective un mode de réalisation d'un dispositif dans lequel un élément de capture peut être dissocié du dispositif,
- La figure 5 représente en perspective un mode de réalisation d'un dispositif comportant deux flux latéraux,
- Les figures 6A et 6B représentent en vue de dessus et en éclaté un mode de réalisation d'une enceinte renfermant un dispositif,
- Les figures 7A et 7B représentent en vue de dessus un autre mode de réalisation d'une enceinte renfermant un dispositif,
- Les figures 8A et 8B représentent en vue de dessus et en éclaté un mode de réalisation d'une enceinte renfermant un dispositif à double flux latéral,
- Les figures 9A et 9B représentent en perspective et en éclaté un système comportant une enceinte et une capsule porte - élément de capture,
- Les figures 10A et 10B montrent la comparaison des profils de spectre MS sur silicium nanoporeux entre le début et la fin de la migration du sérum sur bandelette. Seuil de détection : S/N (signal sur bruit) d'au moins 3,
- La figure 11 montre la comparaison des profils de sérum sur silicium nanoporeux à J0 (ligne A), J21 à température ambiante (ligne B) et J21 à 37°C (ligne C),
- La figure 12 montre la corrélation des qualités de spectre obtenues sur 21 jours entre les conditions de stockage à température ambiante et à 37°C. Biais étuve/RT : Répartition des corrélations de chaque pic détecté (intensités, S/N) entre 21 jours à R.T. et 21 jours à 37°C. Réplica analytique : Répartition des corrélations de chaque pic détecté entre réplica analytique de mêmes conditions,
- La figure 13 montre la corrélation des profils (mêmes pics détectés, intensités, signal/bruit) entre J0 et J21 à 37°C (panneau A) et entre J0 et J21 à température ambiante (panneau B).

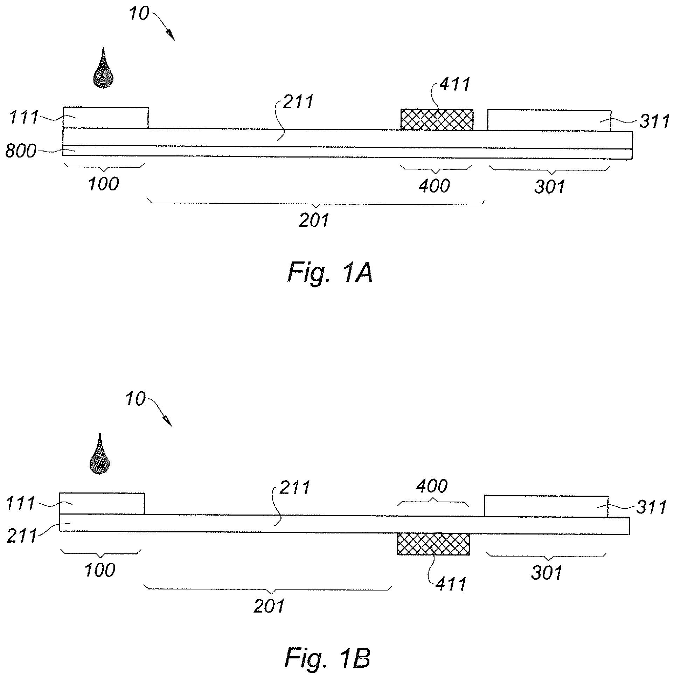

Le dispositif à flux latéral 10 illustré en figure 1A comporte une languette de matériau drainant 211 reposant sur un support 800. Ce matériau drainant est de préférence non fibreux, la taille des pores étant comprise entre 0,2 µm et 1 µm, voire entre 0,2 µm et 10 µm. Il peut s'agir de Nitrocellulose, d'Acrylique, de Polyéthylène Sulfone, de PVDF, PTFE, Nitrate de Cellulose...

De son côté, le support 800 est réalisé en un matériau étanche, prévu pour apporter une certaine rigidité au matériau drainant, par exemple Polypropylène, Vinyl W^{™}, PVC (polyvinyl chloride), HIPS (high impact polystyrene) polyester... Néanmoins ce support n'est pas indispensable, le matériau drainant pouvant posséder une rigidité suffisante. La figure 1B illustre un dispositif dont le matériau drainant ne comporte pas de support.

Le dispositif 10 possède successivement :
- à une première extrémité, une zone de réception 100, comportant un premier matériau absorbant 111 disposé sur et directement en contact avec une face du matériau drainant, prévu pour recevoir un échantillon de liquide ; cette face sera par la suite appelée face supérieure du matériau drainant, la face opposée étant appelée face inférieure,
- une zone de migration 201 du liquide,
- à une seconde extrémité, une zone réservoir 301, comportant un second matériau absorbant 311 disposé sur et entièrement en contact avec la face supérieure du matériau drainant, prévu pour absorber l'excès de liquide ayant parcouru le matériau drainant.

De cette façon, le premier matériau absorbant, le matériau drainant et le second matériau absorbant sont en continuité fluidique : le liquide déposé sur le premier matériau absorbant 111 pénètre par capillarité dans le matériau drainant 211 et progresse le long de ce matériau drainant vers le second matériau absorbant 311. Comme il sera vu plus loin, d'autres dispositions permettent de garantir cette continuité fluidique.

Les premier et second matériaux absorbants 111, 311 sont par exemple réalisés en fibre de cellulose, fibre de verre, Polyvinyle Sulfone... Ces matériaux présentent de préférence une structure désordonnée (fibres) ou ordonnée (maillage), de façon à permettre une filtration du liquide, dans la zone de réception 100, en retenant notamment des organismes et débris cellulaires dans le cas, par exemple, de liquides biologiques comme le sang (érythrocytes, leucocytes, plaquettes) ou l'urine (débris, bactéries), en amont de la zone de migration 201. Sur un même dispositif, ces premier et second matériaux absorbants peuvent être identiques ou différents.

Le premier matériau absorbant 111 peut avoir subi un prétraitement par imprégnation dans une solution puis séchage, de manière à permettre un conditionnement du liquide au niveau de la zone de réception 100, par exemple modification du pH, ajouts d'ions, ajouts de calibrant internes pour l'analyse moléculaire quantitative, anticoagulants, stabilisants, anti-protéases...

Le volume du second matériau absorbant 311 est généralement supérieur aux volumes respectifs du premier matériau absorbant 111 et du matériau drainant 211, de manière à ce que sa capacité d'absorption soit supérieure à la capacité d'absorption du premier matériau absorbant 111 et du matériau drainant 211. Ainsi, la zone réservoir 301 fait office de pompe capillaire, permettant une circulation fluidique entre la zone de réception 100 et la zone réservoir 301.

La zone de migration 201 comporte une zone de capture 400 ; tel que représenté les figures 1A et 1B, un élément de capture 411 est disposé sur cette zone de capture, en contact avec le matériau drainant 211. Comme illustré, l'élément de capture 411 peut être disposé sur la face supérieure ou sur la face inférieure du matériau drainant 211.

L'élément de capture 411 est prévu pour capturer un ou des analytes d'intérêt au cours de leur migration dans le matériau drainant de la zone de réception vers la zone réservoir.

Dans les modes de réalisation illustrés, l'élément de capture 411 est réalisé en Silicium nanoporeux ou mésoporeux ; comme illustré à l'exemple 2 ci-après, ce matériau présente l'avantage de conserver les analytes d'intérêt plusieurs jours sans dégradation.

L'élément de capture 411 est détachable du matériau drainant 211 pour permettre une analyse quantitative du ou des analytes d'intérêt tels que protéines, métabolites, molécules organiques...

Dans un premier mode de réalisation, l'élément de capture 411 est entièrement mobile, c'est-à-dire qu'il est appliqué par l'opérateur sur la zone de capture 400 du matériau drainant 211 au moment du prélèvement puis il en est détaché pour être envoyé dans un centre d'analyse pour dosage du ou des analytes d'intérêt. Dans d'autres modes de réalisation, l'élément de capture est solidaire du dispositif, ce dernier n'étant détaché du matériau drainant qu'au moment du dosage quantitatif.

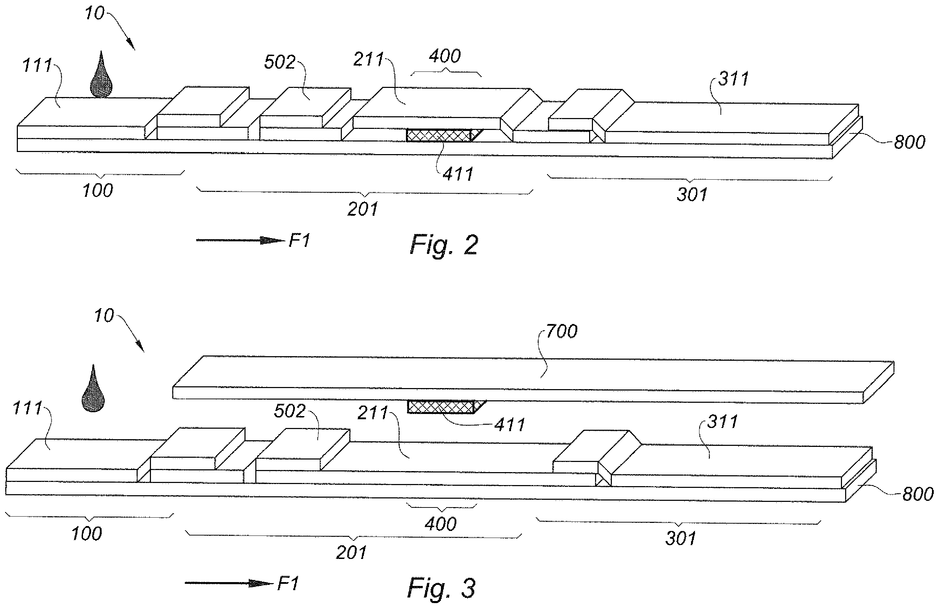

La figure 2 illustre un autre mode de réalisation du dispositif 10 dans lequel :
- l'élément de capture 411 est maintenu entre le support 800 et le matériau drainant 211, il est donc en contact avec la face inférieure du matériau drainant ; dans ce mode de réalisation, le dispositif 10 comporte dès l'origine l'élément de capture 411,
- la zone de réception comprend un matériau de transfert 502 qui s'intercale entre le premier matériau absorbant 111 et le matériau drainant 211, tout en assurant la continuité fluidique entre ces deux matériaux ; ce matériau de transfert permet par exemple la filtration des éléments figurés du sang ; il est réalisé en un matériau fibreux, définissant une maille, ordonnée ou non, dont la taille moyenne est inférieure à la taille des mailles du premier matériau absorbant 111, pour permettre un pompage capillaire du liquide provenant de ce premier matériau.

Dans la réalisation pratique, le premier matériau absorbant 111 chevauche partiellement le matériau de transfert 502, qui lui-même chevauche partiellement le matériau drainant 211. A l'autre extrémité du dispositif, le second matériau absorbant 311 chevauche partiellement le matériau drainant 211. On assure ainsi la continuité fluidique entre la zone de réception 100 et la zone réservoir 301. Le liquide progresse par capillarité dans le sens de la flèche F1.

La figure 3 illustre un autre mode de réalisation du dispositif 10 comportant un matériau de couverture 700 recouvrant partiellement le premier matériau absorbant 111, le matériau de transfert 502, le matériau drainant 211 et le second matériau absorbant 311. Un élément de capture 411 est disposé entre le matériau de couverture et le matériau drainant 211, il est donc maintenu sur la face supérieure de ce matériau drainant.

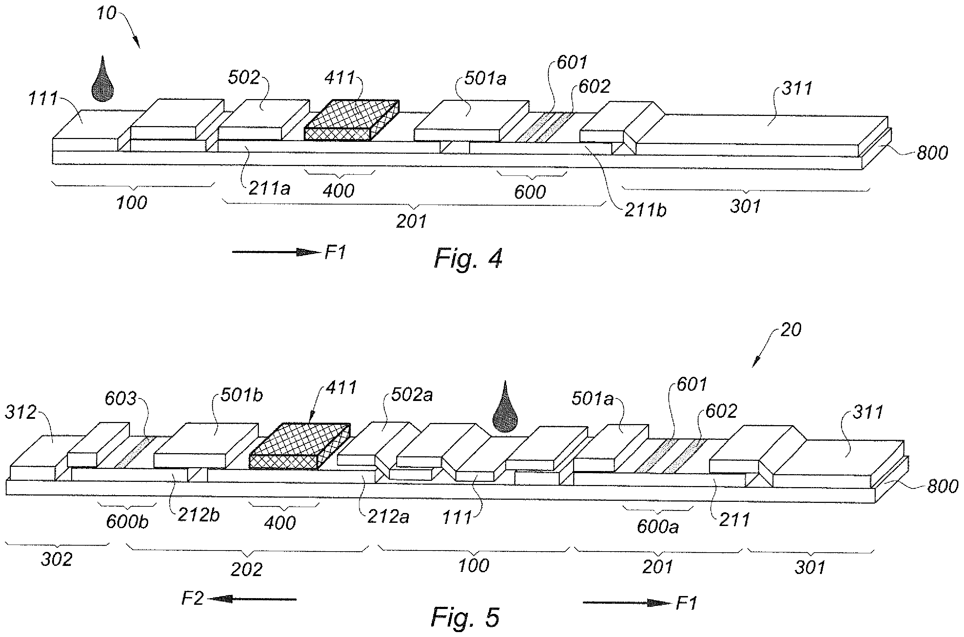

La figure 4 illustre un autre mode de réalisation du dispositif 10. Dans ce mode de réalisation, le matériau drainant comporte deux segments séparés, un segment amont 211a et un segment aval 211b. Le dispositif comporte en outre un matériau de conjugaison 501a à cheval sur ces deux segments afin d'une part de forcer le liquide à traverser le matériau de conjugaison 501a et d'autre part d'assurer la continuité fluidique entre la zone de réception 100 et la zone réservoir 301. Le segment aval 211b de matériau drainant comporte une zone de détection 600 incluant un motif de détection constitué de deux lignes 601 et 602 destinées à détecter la présence ou non d'un analyte ciblé, et à révéler la migration totale du liquide pour valider le test. L'élément de capture 411 est disposé sur le premier segment 211a de matériau drainant, en amont du matériau de conjugaison 501a.

La figure 5 illustre un autre mode de réalisation du dispositif 20 comportant deux zones de migration 201, 202 situées de part et d'autre d'une zone de réception 100 et deux zones réservoir 301, 302 aux extrémités respectives des zones de migration. Ce dispositif comporte donc un premier et un second flux latéraux en sens opposés, représentés par les flèches F1 et F2 de la figure 5.

Le premier flux latéral F1 comporte, comme décrit précédemment, en continuité fluidique avec le premier matériau absorbant 111 et d'amont en aval dans le sens de la flèche F1, un premier matériau de conjugaison 501a, un premier matériau drainant 211 en contact avec le premier matériau de conjugaison, ce premier matériau drainant comportant une première zone de détection 600a et un second matériau absorbant 311 formant premier réservoir.

Le second flux latéral F2 comporte, en continuité fluidique avec le premier matériau absorbant 111 et d'amont en aval dans le sens de la flèche F2, un matériau de transfert 502a, un second matériau drainant sous la forme d'un premier segment 212a et d'un second segment 212b de matériau drainant ; un élément de capture 411 est disposé sur le premier segment ; un second matériau de conjugaison 501b est disposé à cheval sur les deux segments ; le second segment 212b comporte une seconde zone de détection 600b. Un second matériau absorbant 312 formant second réservoir est finalement en continuité fluidique avec le second segment 212b.

Le second matériau de conjugaison 501b comporte des secondes particules de conjugaison aptes à se fixer sur un analyte que l'on est certain de trouver dans le liquide. De cette façon, la détection de ces secondes particules de conjugaison dans la seconde zone de détection 600b signifie que le liquide a au moins franchi le premier segment 212a de matériau drainant et qu'en conséquence l'élément de capture 411 a pu capturer le ou les analyte(s) d'intérêt.

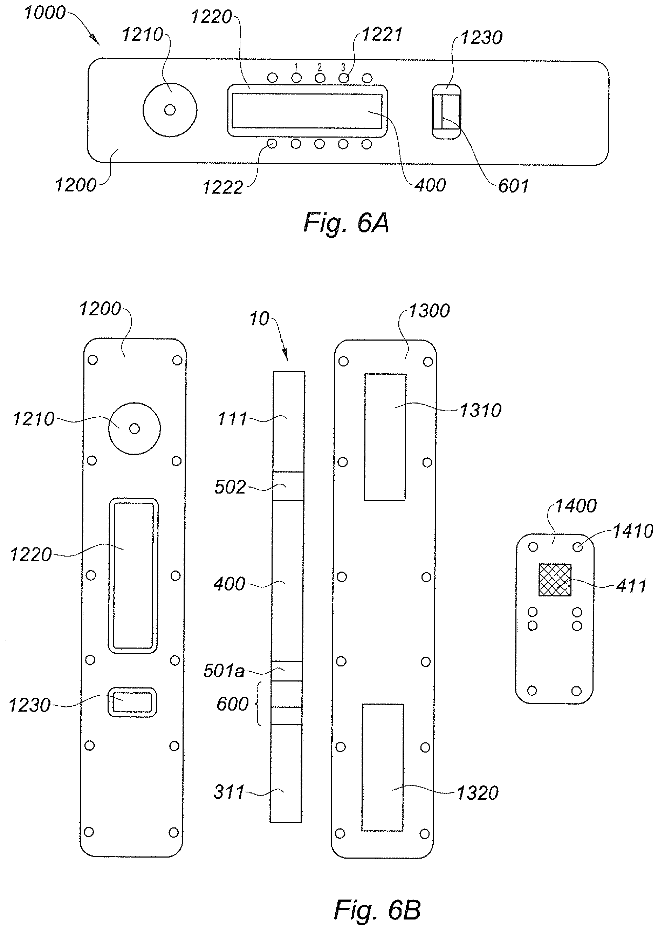

Les figures 6A et 6B représentent un système de prélèvement comprenant un dispositif 10 et une enceinte 1000 renfermant le dispositif.

L'enceinte se présente sous la forme d'une baguette comportant une partie supérieure 1200 et une partie inférieure 1300, ces deux parties étant réunies, en utilisation, par exemple par clipage et/ou collage ; lorsque ces deux parties sont réunies, elles enserrent le dispositif 10. La figure 6A représente la face externe de la partie supérieure 1200, la figure 6B représente le dispositif ouvert, c'est à dire les faces internes des parties supérieure et inférieure.

La partie supérieure 1200 comporte successivement :
- une première ouverture 1210, qui se trouve en vis-à-vis du premier matériau absorbant 111 du dispositif ; cette première ouverture est destinée à recevoir l'échantillon de liquide et le transmettre au premier matériau absorbant,
- une deuxième ouverture 1220 de forme allongée, en vis-à-vis du matériau drainant 211,
- une première fenêtre 1230, en vis-à-vis de la zone de détection 600 du dispositif; cette fenêtre peut être ouverte et former une troisième ouverture, ou comporter un matériau translucide permettant la lecture d'un signal de détection 601.

La partie inférieure 1300 comporte des logements en creux 1310, 1320 prévus pour recevoir et maintenir le dispositif 10.

Le système comprend en outre une plaquette 1400 comportant un élément de capture 411 sur une de ses faces. La plaquette est prévue pour se fixer au moins temporairement, par exemple par clipage, sur les bords de la deuxième ouverture 1220 de façon que l'élément de capture vienne au contact du matériau drainant 211 au travers de l'a deuxième ouverture 1220. A cet effet, la partie supérieure 1200 de l'enceinte et la plaquette 1400 comportent des moyens 1222, 1410 qui coopèrent, par exemple par clipage, pour fixer la plaquette sur la partie supérieure de l'enceinte de façon réversible, pendant le temps du prélèvement. Après le prélèvement, seule cette plaquette 1400 avec son élément de capture est envoyée au centre d'analyse.

La partie supérieure 1200 de l'enceinte 1000 comporte également des graduations 1221 disposées le long de la deuxième ouverture 1220 pour guider l'utilisateur dans le positionnement de la plaquette 1400 et donc de l'élément de capture 411 sur le matériau drainant 211, ceci en fonction du ou des analytes d'intérêt recherchés.

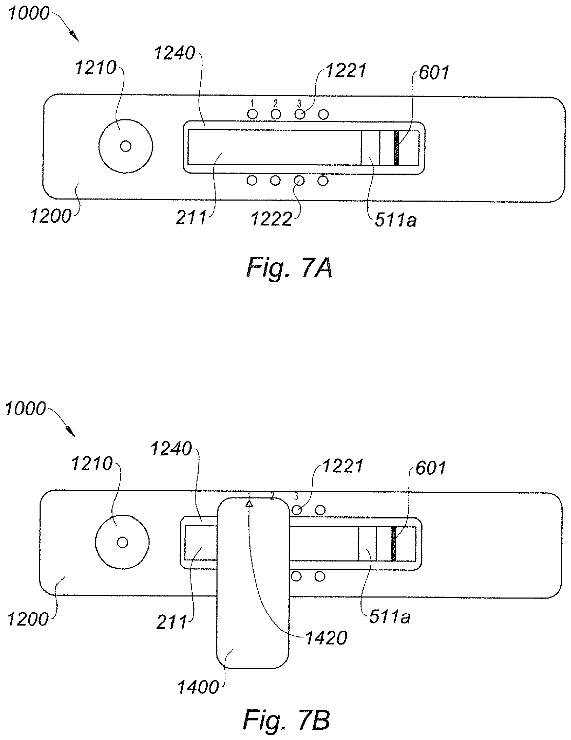

La figure 7A illustre une variante de l'enceinte 1000 dans laquelle la deuxième ouverture et la première fenêtre sont fusionnées en une ouverture unique 1240, ce qui rend apparent le matériau de conjugaison 511a.

La figure 7B illustre une plaquette 1400 clipée sur la partie supérieure 1200 de l'enceinte 1000 ; comme il a été vu plus haut, la plaquette comporte sur la face non visible de la figure 7B un élément de capture 411 qui se trouve ainsi mis en contact avec le matériau drainant 211 en vue d'un prélèvement d'analyte. La face visible de la plaquette 1400 comporte un repère ou ergot 1420 qui est disposé en regard d'une graduation 1221 de la partie supérieure 1200.

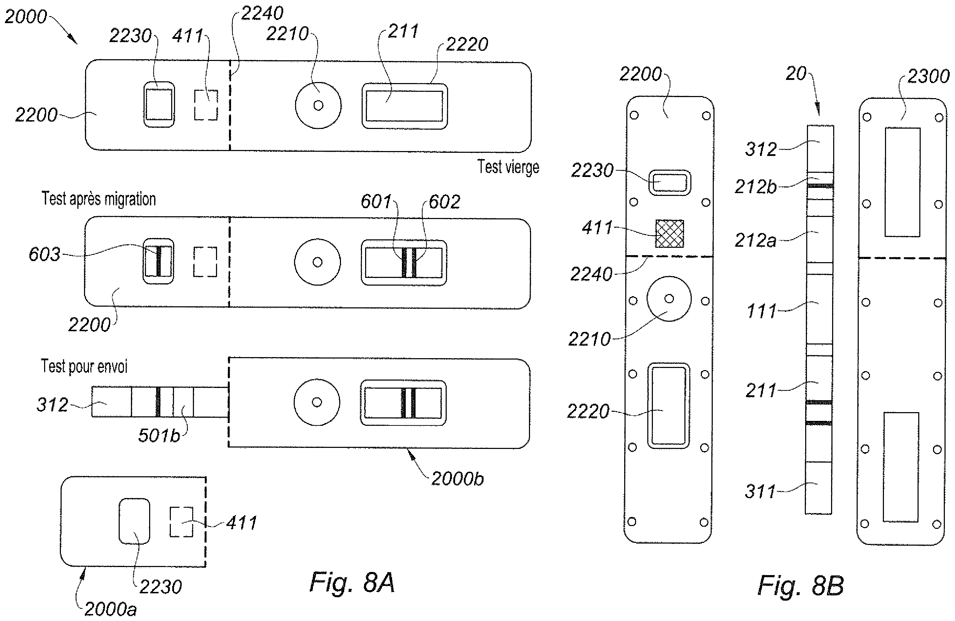

Les figures 8A et 8B représentent un autre mode de réalisation d'une enceinte 2000 adaptée au dispositif 20 à double flux illustré en figure 5. Comme précédemment, l'enceinte se présente sous la forme d'une baguette comportant une partie supérieure 2200 et une partie inférieure 2300, ces deux parties étant réunies, en utilisation, par exemple par clipage ou collage ; lorsque ces deux parties sont réunies, elles enserrent le dispositif 20. La figure 8A représente plusieurs vues de la face externe de la partie supérieure 2200, la figure 6B représente le dispositif ouvert, c'est à dire les faces internes des parties supérieure et inférieure.

La partie supérieure 2200 comporte :
- une première ouverture 2210, qui se trouve en vis-à-vis du premier matériau absorbant 111 du dispositif ; cette première ouverture est destinée à recevoir l'échantillon de liquide et le transmettre au premier matériau absorbant, et de part et d'autre de cette première ouverture :
- une première fenêtre 2220, en vis-à-vis de la zone de détection 600a du matériau drainant 211 ; cette fenêtre peut être ouverte et former une deuxième ouverture, ou comporter un matériau translucide permettant la lecture de signaux de détection 601, 602,
- une deuxième fenêtre 2230, en vis-à-vis de la zone de détection 600b du second segment de matériau drainant 212b ; cette fenêtre peut être ouverte et former une troisième ouverture, ou comporter un matériau translucide permettant la lecture du signal de détection 603.

Dans le mode de réalisation illustré, l'enceinte 2000 comporte également une ligne de découpe ou partie fragile 2240 située entre la première ouverture 2210 et la deuxième fenêtre 2230, permettant de scinder facilement l'enceinte 2000 en deux parties, une première partie sécable 2000a, comportant l'élément de capture 411, et une seconde partie 2000b.

Comme il est visible sur la figure 8B, la partie supérieure 2200 de l'enceinte comporte, sur sa face intérieure, un élément de capture 411 situé entre la ligne de découpe 2240 et la deuxième fenêtre 2230, visible par transparence en figure 8A. Lorsque l'enceinte renferme un dispositif 20, l'élément de capture 411 vient au contact d'un premier segment de matériau drainant 212a.

De cette façon, après prélèvement du ou des analytes d'intérêt, la première partie sécable 2000a, comportant l'élément de capture 411, est séparée de la seconde partie 2000b de l'enceinte, et seule la première partie est envoyée au centre d'analyse pour dosage des analytes.

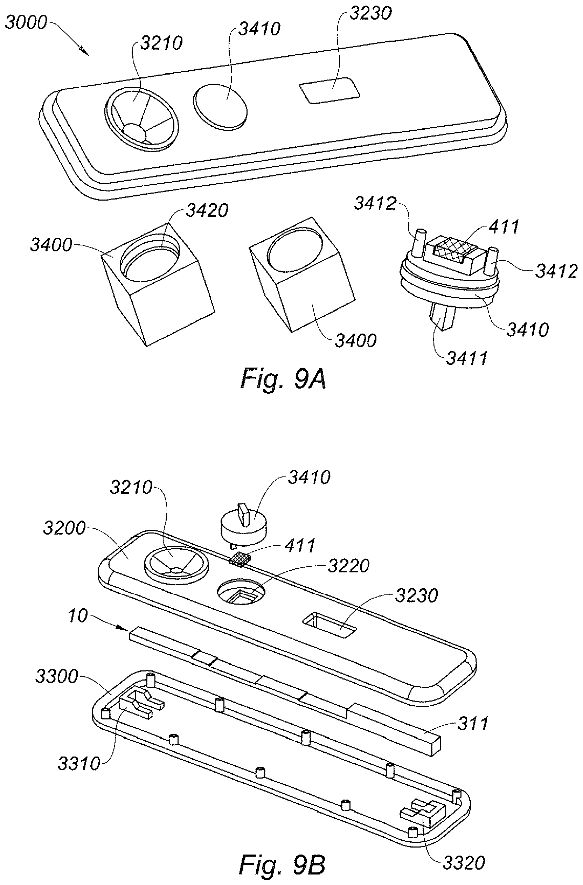

Les figures 9A et 9B illustrent une variante du système de prélèvement comportant une enceinte 3000 renfermant un dispositif 10 et une capsule 3400.

L'enceinte 3000 est semblable à l'enceinte 1000 des figures 6A et 6B. Elle comprend une partie supérieure 3200 et une partie inférieure 3300 qui, lorsqu'elles sont réunies, enserrent un dispositif 10.

La partie supérieure 3200 comporte successivement :
- une première ouverture 3210, qui se trouve en vis-à-vis du premier matériau absorbant 111 du dispositif,
- une deuxième ouverture 3220 de forme circulaire, en vis-à-vis du matériau drainant 211 qui sera détaillée plus loin,
- une première fenêtre 3230, en vis-à-vis de la zone de détection 600 du dispositif.

La partie inférieure 1300 comporte des logements en creux 3310, 3320 prévus pour recevoir et maintenir le dispositif 10.

Le système comprend en outre un récipient ou capsule 3400 comportant un bouchon 3410. Ce bouchon comporte sur une face dite supérieure une poignée de manœuvre 3411, et sur une face dite inférieure :
- deux picots ou ergots 3412, de sorte que le bouchon 3410 est apte à se fixer (par vissage, clipage, emboîtement ou autre) à la fois sur une ouverture 3420 de la capsule et sur la deuxième ouverture 3220 de l'enceinte 3000, les modes de fixation sur le récipient 3400 et sur l'enceinte 3000 pouvant être différents,
- un élément de capture 411.

Dans ce mode de réalisation, l'élément de capture 411 est livré enfermé dans la capsule 3400, il en est ôté avec le bouchon 3410, ce bouchon est fixé sur l'enceinte 3000 pour le temps du prélèvement. Lorsque le prélèvement est achevé, le bouchon est détaché de l'enceinte et replacé sur la capsule 3400. La capsule est envoyée seule au centre d'analyse. L'avantage est que l'opérateur n'a jamais à toucher l'élément de capture 411 qui reste donc à l'abri de toute contamination.

Cette description n'est pas limitative. Ainsi :
- le dispositif pourrait ne pas être rectiligne comme représenté dans les figures ; notamment, dans le mode de réalisation à deux flux latéraux, ces deux flux pourraient partager la même zone réservoir,
- l'enceinte ne comporte pas nécessairement de ligne de découpe 2240, l'enceinte dans son entier peut être envoyée au laboratoire,
- bien que les figures 6A à 9B n'illustrent que des systèmes dans lesquels l'élément de capture 411 est détachable de l'enceinte 1000, 2000, 3000, ces systèmes pourraient comporter un dispositif dans lequel l'élément de capture n'est pas facilement détachable du matériau drainant, comme illustré en figures 2 et 3.

Les exemples expérimentaux suivants illustrent certains aspects l'invention sans toutefois limiter son étendue.

### Exemple 1 : Comparaison des profils de spectre MS sur silicium nanoporeux entre le début et la fin de la migration du serum sur bandelette

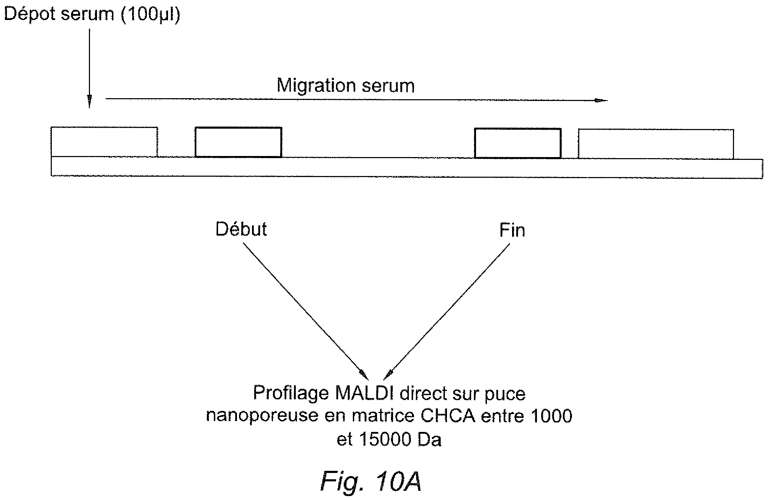

Les conditions d'expérimentation sont décrites dans le schéma présenté en figure 10A. Brièvement, deux puces de silicium nanoporeux de même dimension (5x5x0.75mm, 1µm d'épaisseur de porosification en surface, pores de 20nm de section et taux de porosité de l'ordre de 50%) ont été déposées sur une bande de nitrocellulose, l'une accolée juste après la zone de réception 111 (zone A) et l'autre accolée juste avant le matériau absorbant 311 (zone B) de la zone de réception. Après dépôt de 100 µl de sérum et migration jusqu'à la zone réservoir, les deux puces ont été récupérées, rincées 3 fois à l'eau avant de réaliser une analyse par MALDI directement sur la puce, en matrice CHCA, entre 1000 et 15000 Da.

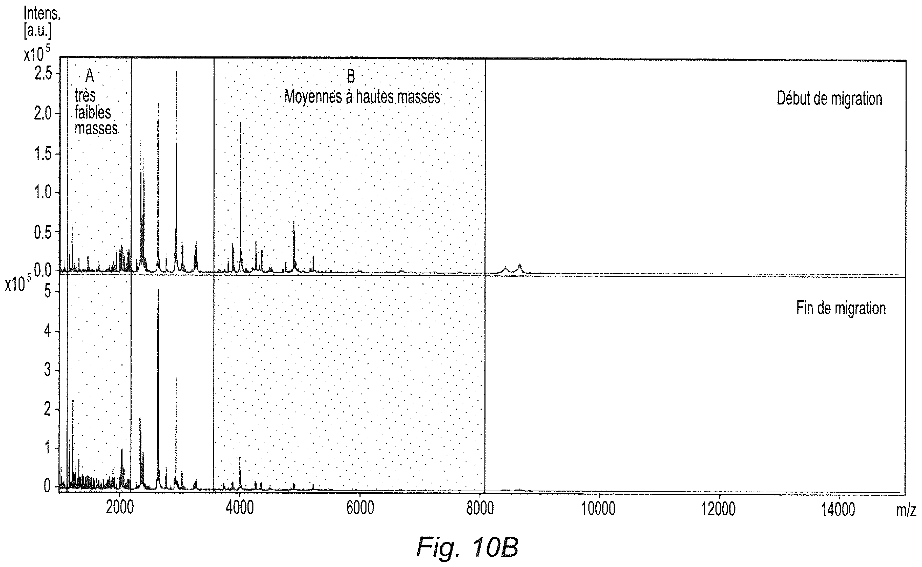

Les résultats, illustrés sur la figure 10B et dans le tableau 1 ci-dessous, suggèrent un effet de séparation des protéines par chromatographie d'exclusion stérique pendant la migration du sérum sur la nitrocellulose. En effet, on observe sur le poreux en début de migration moins de pics (46) avec un haut rapport signal/bruit sur les masses inférieures à 2400 Da par rapport au poreux en fin de migration (68). Cette observation s'inverse pour les masses supérieures à 3500 Da. Le maillage de la nitrocellulose agit donc comme une surface chromatographique, enrichissant les molécules de plus bas poids moléculaire pendant la migration.

**Tableau 1**

| | Nombre de pics détectés avec un haut signal / bruit | |
|---|---|---|
| | Zone de masse A (1000 - 2200 Da) | Zone de masse B (3500 - 9000 Da) |
| Elément de capture (poreux) en début de migration | 46 | 31 |
| Elément de capture (poreux) en fin de migration | 68 | 20 |

La détection MALDI étant sensible à ces enrichissements spécifiques, les rapports signal/bruit pour chaque gamme de masse sont changés, ainsi que leur détection spectrale.

### Exemple 2 : Etude de la stabilisation des molécules piégées dans le silicium nanoporeux sur 21 jours

Les puces de silicium nanoporeux utilisées ici sont identiques à celles décrites dans l'exemple 1. Dix microlitres de sérum ont été déposés directement sur chaque puce, puis les étapes suivantes ont été réalisées :
- incubation du sérum sur poreux : 10min,
- rinçage 3x à l'eau distillée
- stockage en sachet avec du dessicant (gel de silice) à température ambiante et à 37°C (humidité atmosphérique).
- profilage MALDI direct en CHCA entre 1000 et 15000Da tous les 7 jours.

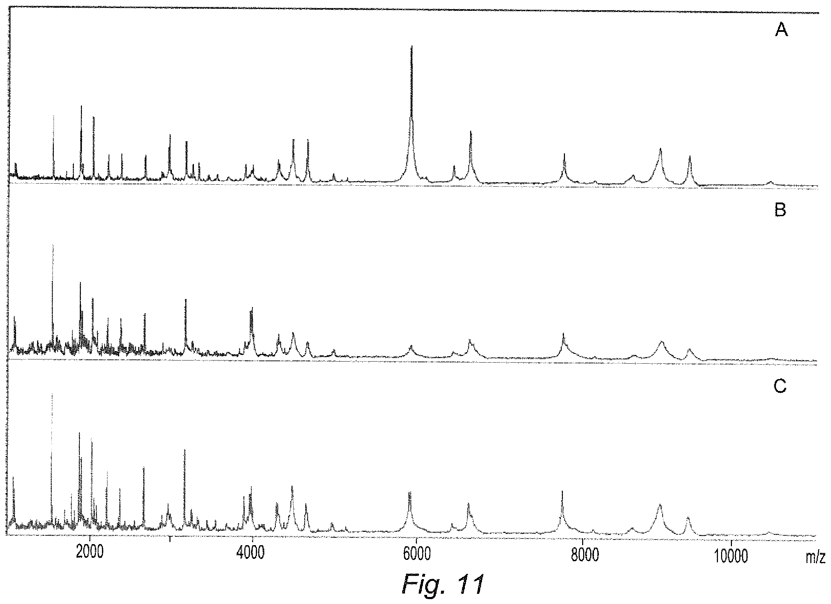

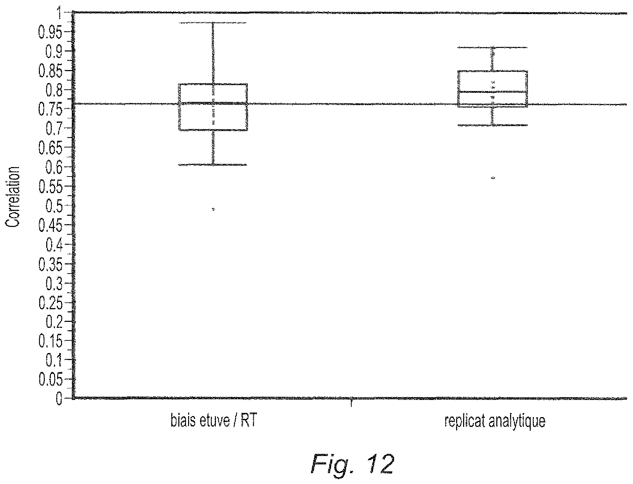

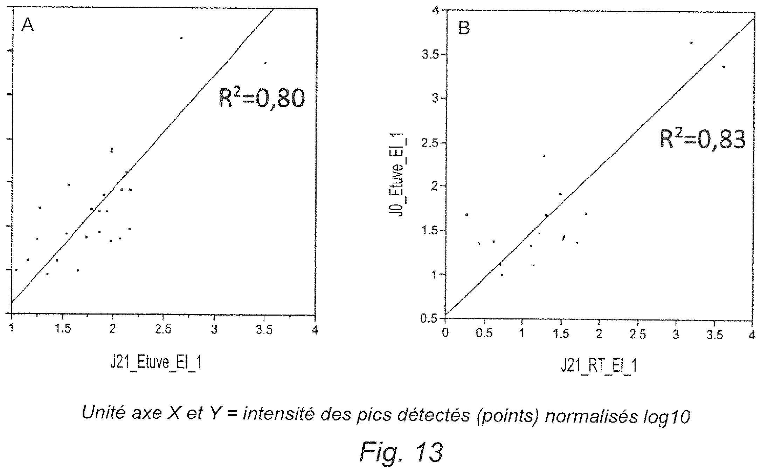

Les résultats, illustrés à la figure 11, montrent que les profils sont comparables en termes d'espèces détectées. Aucun produit de dégradation n'est observable. La corrélation des qualités de spectre entre les différentes conditions de stockage (figure 12), ainsi que la corrélation des profils entre J0 et J21 (à température ambiante ou à 37°C, figure 13) est supérieure à 0,75.

Sur la figure 12, l'unité de l'axe des Y est l'indice de corrélation, la valeur 1 étant une corrélation parfaite.

Sur la figure 13, l'unité des axes des X et des Y est l'intensité des pics détectés (points) normalisée en log10.

## Revendications

1. Dispositif à flux latéral (10, 20) pour le prélèvement d'au moins un analyte d'intérêt dans un liquide, comportant :
- une zone de réception (100) du liquide, comportant un premier matériau absorbant (111) apte à recueillir le liquide,
- au moins une zone de migration (201, 202) du liquide, s'étendant entre une première extrémité et une deuxième extrémité, chaque zone de migration comportant un matériau drainant (211, 211a, 211b, 212a, 212b) en continuité fluidique avec le premier matériau absorbant, le matériau drainant étant apte à collecter le liquide absorbé par la zone de réception et à drainer le liquide de sa première extrémité vers sa deuxième extrémité,
- au moins une zone réservoir (301, 302), comportant un deuxième matériau absorbant (311, 312), en contact avec le matériau drainant au niveau de la deuxième extrémité de chaque zone de migration, le deuxième matériau absorbant étant apte à absorber le liquide drainé par le matériau drainant,
- des moyens de détection entre la zone de réception et la zone réservoir, permettant de déterminer la présence ou l'absence dudit/desdits analyte(s) d'intérêt dans l'échantillon,
**caractérisé en ce qu'**au moins l'une desdites zones de migration comporte également au moins une zone de capture (400), le dispositif comprenant en outre un élément de capture (411) appliqué sur chaque zone de capture (400), contre le matériau drainant entre sa première et sa deuxième extrémité, chaque élément de capture (411) comportant un matériau nanoporeux ou mésoporeux en silicium et étant apte à capturer un analyte d'intérêt présent dans le liquide et **en ce que** chaque élément de capture (411) est apte à être séparé du matériau drainant (211, 211a, 212a).

2. Dispositif à flux latéral selon la revendication 1, **caractérisé en ce que** les moyens de détection comportent :
- au moins un matériau de conjugaison (501a, 501b) disposé au contact du matériau drainant (211a, 211b, 212a, 212b), ledit matériau de conjugaison comportant des particules de conjugaison aptes à se lier au(x) analyte(s) d'intérêt,
- un matériau apte à immobiliser les particules de conjugaison liées à un analyte d'intérêt et à les révéler, ledit matériau imprégnant une zone de détection (600, 600a, 600b) du matériau drainant.

3. Dispositif à flux latéral (20) selon l'une quelconque des revendications précédentes, **caractérisé en ce qu'**il comporte une première (201) et une seconde (202) zones de migration comportant respectivement un premier (211, 211a, 211b) et un second (212a, 212b) matériau drainant, la zone de réception (100) étant située entre lesdites deux zones de migration

4. Dispositif à flux latéral selon l'une des revendications précédentes, **caractérisé en ce que** le premier (111) et/ou le second des matériaux absorbants (311, 312) sont réalisés dans un matériau fibreux.

5. Dispositif à flux latéral selon l'une des revendications précédentes, **caractérisé en ce que** la zone de réception (100) comporte en outre un matériau de transfert (502).

6. Dispositif à flux latéral selon l'une des revendications précédentes, **caractérisé en ce qu'**au moins l'un des matériaux drainant (211, 212) comporte un matériau nanoporeux ou mésoporeux.

7. Dispositif à flux latéral selon l'une des revendications 2 à 6, **caractérisé en ce que** la zone de capture (400) est située en amont du matériau de conjugaison (501a, 501b) dans le sens de drainage (F1, F2) du liquide dans le matériau drainant (211, 211a, 211b, 212a, 212b).

8. Dispositif à flux latéral selon l'une des revendications 2 à 7, **caractérisé en ce que** au moins l'un des matériaux de conjugaison (501a, 501b) comporte des particules de conjugaison aptes à se lier à un analyte que l'on est certain de trouver dans le liquide.

9. Dispositif à flux latéral selon l'une des revendications précédentes, **caractérisé en ce que** le premier matériau absorbant (111), le matériau drainant (211, 212) et le second matériau absorbant (311, 312) sont disposés sur un matériau support (800).

10. Dispositif à flux latéral selon l'une des revendications précédentes, **caractérisé en ce qu'**il comporte en outre un matériau de couverture (700) apte à recouvrir au moins une partie de la zone de réception, la zone de migration et/ou la zone réservoir.

11. Dispositif à flux latéral selon l'une des revendications 1 à 10, **caractérisé en ce que** l'élément de capture (411) est maintenu contre le matériau drainant (211).

12. Système de prélèvement d'analytes d'intérêt dans un liquide, **caractérisé en ce qu'**il comporte une enceinte (1000) renfermant un dispositif à flux latéral (10) selon l'une des revendications précédentes 1 à 11 comportant une seule zone de migration (201), ladite enceinte comportant :
- une première ouverture (1210) disposée en vis-à-vis de la zone de réception (100) dudit dispositif,
- une première fenêtre (1230) disposée en vis-à-vis de la zone de détection (600) afin de permettre de lire un signal de détection (601, 602).

13. Système de prélèvement d'analytes d'intérêt dans un liquide, **caractérisé en ce qu'**il comporte une enceinte (2000) renfermant un dispositif à flux latéral (20) selon l'une des revendications 1 à 11 comportant deux zones de migration (201, 202), ladite enceinte comportant :
- une première ouverture (2210) disposée en vis-à-vis de la zone de réception (100) dudit dispositif,
- une première fenêtre (2220) disposée en vis-à-vis de la première zone de détection (600a) afin de permettre de lire un premier signal de détection (601, 602),
- une seconde fenêtre (2230) disposée en vis-à-vis de la seconde zone de détection (600b) afin de permettre de lire un second signal de détection (603).

14. Système de prélèvement d'analytes d'intérêt dans un liquide selon la revendication 12 ou 13, **caractérisé en ce que** l'enceinte (1000, 2000) comporte en outre une deuxième ouverture (1220) disposée en vis-à-vis de la zone de capture (400) du dispositif, de façon à pouvoir déposer un élément de capture (411) sur ladite zone de capture et/ou l'en retirer.

15. Système de prélèvement d'analytes d'intérêt dans un liquide selon l'une des revendications 12 à 14, **caractérisé en ce que** le dispositif (10, 20) comprend en outre au moins un élément de capture (411) maintenu sur la zone de capture (400), ledit au moins un élément de capture étant apte à capturer un analyte d'intérêt présent dans le liquide.

16. Système de prélèvement d'analytes d'intérêt dans un liquide selon l'une des revendications 12 à 15, **caractérisé en ce que** l'élément de capture (411) est fixé à un support détachable de l'enceinte.

17. Système de prélèvement d'analytes d'intérêt dans un liquide selon la revendication 16, **caractérisé en ce que** le support détachable de l'enceinte est l'un des éléments suivants :
- une plaquette (1400) apte à se fixer au moins temporairement sur les bords de la deuxième ouverture (1220),
- une partie sécable (2000a) de l'enceinte (2000),
- un bouchon (3410) apte à se fixer à la fois sur une capsule (3400) et sur la deuxième ouverture (3220).

18. Procédé de prélèvement d'au moins un analyte d'intérêt dans un échantillon, comportant les étapes suivantes :
- déposer ledit échantillon sur la zone de réception d'un dispositif selon l'une quelconque des revendications 1 à 11, et
- attendre que le liquide ait migré jusqu'à la zone réservoir, **caractérisé en ce qu'**en cas de présence de l'analyte d'intérêt dans l'échantillon, avérée par les moyens de détection, ledit analyte est prélevé dans l'élément de capture (411).

19. Procédé de prélèvement selon la revendication 18, **caractérisé en ce que** le dispositif est compris dans un système de prélèvement selon l'une quelconque des revendications 12 à 17 et qu'à l'issue de l'étape de migration du liquide, l'élément de capture (411) est séparé du reste du dispositif.

20. Procédé de dosage d'au moins un analyte d'intérêt dans un échantillon de liquide, comportant une étape de prélèvement dudit analyte par un procédé selon l'une des revendications 18 ou 19, suivie d'une étape de dosage dudit analyte présent dans l'élément de capture.

## Patentansprüche

1. Lateral-Flow-Vorrichtung (10, 20) zur Entnahme mindestens eines Analyten von Interesse aus einer Flüssigkeit, umfassend:
- eine Empfangszone (100) der Flüssigkeit, die ein erstes absorbierendes Material (111) beinhaltet, das imstande ist, die Flüssigkeit zu sammeln,
- mindestens eine Migrationszone (201, 202) der Flüssigkeit, die sich zwischen einem ersten Ende und einem zweiten Ende erstreckt, wobei jede Migrationszone ein drainierendes Material (211, 211a, 211b, 212a, 212b) in fluider Kontinuität mit dem ersten absorbierenden Material beinhaltet, wobei das drainierende Material imstande ist, die absorbierte Flüssigkeit durch die Empfangszone aufzusammeln und die Flüssigkeit von ihrem ersten Ende zu ihrem zweiten Ende zu drainieren,
- mindestens eine Behälterzone (301, 302), die ein zweites absorbierendes Material (311, 312) in Kontakt mit dem drainierenden Material im Bereich des zweiten Endes jeder Migrationszone beinhaltet, wobei das zweite absorbierende Material imstande ist, die drainierte Flüssigkeit durch das drainierende Material zu absorbieren,
- Erkennungsmittel zwischen der Empfangszone und der Behälterzone, die es ermöglichen, die Anwesenheit oder die Abwesenheit des/ der Analyten von Interesse in der Probe zu bestimmen,
**dadurch gekennzeichnet, dass** mindestens eine der Migrationszonen ebenfalls mindestens eine Erfassungszone (400) beinhaltet, wobei die Vorrichtung weiter ein Erfassungselement (411) umfasst, das in jeder Erfassungszone (400) gegen das drainierende Material zwischen ihrem ersten Ende und ihrem zweiten Ende angewendet wird, wobei jedes Erfassungselement (411) ein nanoporöses oder mesoporöses Material aus Silicium beinhaltet und imstande ist, einen Analyten von Interesse zu erfassen, der in der Flüssigkeit vorhanden ist, und dadurch, dass jedes Erfassungselement (411) imstande ist, von dem drainierenden Material (211, 211a, 212a) getrennt zu werden.

2. Lateral-Flow-Vorrichtung nach Anspruch 1, **dadurch gekennzeichnet, dass** die Erkennungsmittel Folgendes beinhalten:
- mindestens ein Konjugationsmaterial (501a, 501b), das in Kontakt mit dem drainierenden Material (211a, 211b, 212a, 212b) angeordnet ist, wobei das Konjugationsmaterial Konjugationspartikel beinhaltet, die imstande sind, sich mit dem (den) Analyten von Interesse zu verbinden,
- ein Material, das imstande ist, die Konjugationspartikel, die mit einem Analyten von Interesse verbunden sind, zu stoppen und sie preiszugeben, wobei das Material eine Erkennungszone (600, 600a, 600b) des drainierenden Materials imprägniert.

3. Lateral-Flow-Vorrichtung (20) nach einem der vorstehenden Ansprüche, **dadurch gekennzeichnet, dass** sie eine erste (201) und eine zweite (202) Migrationszone beinhaltet, die jeweils ein erstes (211, 211a, 211b) und ein zweites (212a, 212b) drainierendes Material beinhalten, wobei die Empfangszone (100) zwischen den beiden Migrationszonen gelegen ist.

4. Lateral-Flow-Vorrichtung nach einem der vorstehenden Ansprüche, **dadurch gekennzeichnet, dass** das erste (111) und/ oder das zweite der absorbierenden Materialien (311, 312) aus einem faserigen Material realisiert sind.

5. Lateral-Flow-Vorrichtung nach einem der vorstehenden Ansprüche, **dadurch gekennzeichnet, dass** die Empfangszone (100) weiter ein Transfermaterial (502) beinhaltet.

6. Lateral-Flow-Vorrichtung nach einem der vorstehenden Ansprüche, **dadurch gekennzeichnet, dass** mindestens eines der drainierenden Materialien (211, 212) ein nanoporöses oder mesoporöses Material beinhaltet.

7. Lateral-Flow-Vorrichtung nach einem der Ansprüche 2 bis 6, **dadurch gekennzeichnet, dass** die Erfassungszone (400) stromaufwärts des Konjugationsmaterials (501a, 501b) in der Drainagerichtung (F1, F2) der Flüssigkeit in dem drainierenden Material (211, 211a, 211b, 212a, 212b) gelegen ist.

8. Lateral-Flow-Vorrichtung nach einem der Ansprüche 2 bis 7, **dadurch gekennzeichnet, dass** das mindestens eine Konjugationsmaterial (501a, 501b) Konjugationspartikel beinhaltet, die imstande sind, sich mit einem Analyten zu verbinden, den man sicher ist, in der Flüssigkeit zu finden.

9. Lateral-Flow-Vorrichtung nach einem der vorstehenden Ansprüche, **dadurch gekennzeichnet, dass** das erste absorbierende Material (111), das drainierende Material (211, 212) und das zweite absorbierende Material (311, 312) auf einem Trägermaterial (800) angeordnet sind.

10. Lateral-Flow-Vorrichtung nach einem der vorstehenden Ansprüche, **dadurch gekennzeichnet, dass** sie weiter ein Abdeckmaterial (700) beinhaltet, das imstande ist, mindestens einen Teil der Empfangszone, der Migrationszone und/ oder der Behälterzone abzudecken.

11. Lateral-Flow-Vorrichtung nach einem der Ansprüche 1 bis 10, **dadurch gekennzeichnet, dass** das Erfassungsmaterial (411) gegen das drainierende Material (211) gehalten wird.

12. System zur Entnahme von Analyten von Interesse aus einer Flüssigkeit, **dadurch gekennzeichnet, dass** es einen abgeschlossenen Raum (1000) beinhaltet, der eine Lateral-Flow-Vorrichtung (10) nach einem der vorstehenden Ansprüche 1 bis 11 einschließt, die eine einzige Migrationszone (201) beinhaltet, wobei der abgeschlossene Raum Folgendes beinhaltet:
- eine erste Öffnung (1210), die gegenüber der Empfangszone (100) der Vorrichtung angeordnet ist,
- ein erstes Fenster (1230), das gegenüber der Erkennungszone (600) angeordnet ist, um es zu ermöglichen, ein Erkennungssignal (601, 602) zu lesen.

13. System zur Entnahme von Analyten von Interesse aus einer Flüssigkeit, **dadurch gekennzeichnet, dass** es einen abgeschlossenen Raum (2000) beinhaltet, der eine Lateral-Flow-Vorrichtung (20) nach einem der Ansprüche 1 bis 11 einschließt, die zwei Migrationszonen (201, 202) beinhaltet, wobei der abgeschlossene Raum Folgendes beinhaltet:
- eine erste Öffnung (2210), die gegenüber der Empfangszone (100) der Vorrichtung angeordnet ist,
- ein erstes Fenster (2220), das gegenüber der ersten Erkennungszone (600a) angeordnet ist, um es zu ermöglichen, ein erstes Erkennungssignal (601, 602) zu lesen,
- ein zweites Fenster (2230), das gegenüber der zweiten Erkennungszone (600b) angeordnet ist, um es zu ermöglichen, ein zweites Erkennungssignal (603) zu lesen.

14. System zur Entnahme von Analyten von Interesse aus einer Flüssigkeit nach Anspruch 12 oder 13, **dadurch gekennzeichnet, dass** der abgeschlossene Raum (1000, 2000) weiter eine zweite Öffnung (1220) beinhaltet, die gegenüber der Erfassungszone (400) der Vorrichtung angeordnet ist, um ein Erfassungselement (411) in der Erfassungszone ablegen und/ oder daraus entfernen zu können.

15. System zur Entnahme von Analyten von Interesse aus einer Flüssigkeit nach einem der Ansprüche 12 bis 14, **dadurch gekennzeichnet, dass** die Vorrichtung (10, 20) weiter mindestens ein Erfassungselement (411) umfasst, das in der Erfassungszone (400) gehalten wird, wobei das mindestens eine Erfassungselement imstande ist, einen Analyten von Interesse zu erfassen, der in der Flüssigkeit vorhanden ist.

16. System zur Entnahme von Analyten von Interesse aus einer Flüssigkeit nach einem der Ansprüche 12 bis 15, **dadurch gekennzeichnet, dass** das Erfassungselement (411) an einem abnehmbaren Träger des abgeschlossenen Raumes befestigt ist.

17. System zur Entnahme von Analyten von Interesse aus einer Flüssigkeit nach Anspruch 16, **dadurch gekennzeichnet, dass** der abnehmbare Träger des abgeschlossenen Raumes eines der folgenden Elemente ist:
- ein Plättchen (1400), das imstande ist, sich mindestens vorübergehend an den Rändern der zweiten Öffnung (1220) zu befestigen,
- ein abtrennbarer Teil (2000a) des abgeschlossenen Raumes (2000),
- ein Stopfen (3410), das imstande ist, sich sowohl an einer Kapsel (3400) und auf der zweiten Öffnung (3220) zu befestigen.

18. Verfahren zur Entnahme mindestens eines Analyten von Interesse aus einer Probe, die folgenden Schritte beinhaltend:
- Ablegen der Probe in der Empfangszone einer Vorrichtung nach einem der Ansprüche 1 bis 11, und
- Warten, bis die Flüssigkeit bis zur Behälterzone gewandert ist, **dadurch gekennzeichnet, dass** im Fall des Vorhandenseins des Analyten von Interesse in der Probe, das sich durch die Erkennungsmittel herausgestellt hat, das Analyt in dem Erfassungselement (411) entnommen wird.

19. Verfahren zur Entnahme nach Anspruch 18, **dadurch gekennzeichnet, dass** die Vorrichtung in einem System zur Entnahme nach einem der Ansprüche 12 bis 17 enthalten ist, und dass am Ende des Wanderungsschrittes der Flüssigkeit das Erfassungselement (411) vom Rest der Vorrichtung getrennt wird.

20. Verfahren zur Dosierung mindestens eines Analyten von Interesse in einer Flüssigkeitsprobe, einen Schritt der Entnahme des Analyten durch ein Verfahren nach einem der Ansprüche 18 oder 19 beinhaltend, gefolgt von einem Schritt der Dosierung des in dem Erfassungselement vorhandenen Analyten.

## Claims

1. A lateral flow device (10, 20) for sampling at least one analyte of interest in a liquid, including:
- a reception zone (100) for the liquid, including a first absorbent material (111) capable of collecting the liquid,
- at least one liquid migration zone (201, 202) extending between a first end and a second end, each migration zone including a draining material (211, 211a, 211b, 212a, 212b) in fluid continuity with the first absorbent material, the draining material being capable of collecting the liquid absorbed by the reception zone and of draining the liquid from the first end thereof to the second end thereof,
- at least one reservoir zone (301, 302), including a second absorbent material (311, 312), in contact with the draining material at the second end of each migration zone, the second absorbent material being capable of absorbing the liquid drained by the draining material,
- detection means between the reception zone and the reservoir zone, allowing determining the presence or absence of said analyte(s) of interest in the sample,
**characterized in that** at least one of said migration zones also includes at least one capture zone (400), the device further comprising a capture element (411) applied on each capture zone (400), against the draining material between the first and second ends thereof, each capture element (411) including a nanoporous or mesoporous silicon material and being capable of capturing an analyte of interest present in the liquid and **in that** each capture element (411) is capable of being separated from the draining material (211, 211a, 212a).

2. The lateral flow device according to claim 1, **characterized in that** the detection means include:
- at least one conjugation material (501a, 501b) disposed in contact with the draining material (211a, 211b, 212a, 212b), said conjugation material including conjugation particles capable of binding to the analyte(s) of interest,
- a material capable of immobilising the conjugation particles bound to an analyte of interest and of revealing them, said material impregnating a detection zone (600, 600a, 600b) of the draining material.

3. The lateral flow device (20) according to any one of the preceding claims, **characterized in that** it includes first (201) and second (202) migration zones including respectively first (211, 211a, 211b) and second (212a, 212b) draining materials, the reception zone (100) being located between said two migration zones

4. The lateral flow device according to one of the preceding claims, **characterized in that** the first (111) and/or the second of the absorbent materials (311, 312) are made of a fibrous material.

5. The lateral flow device according to one of the preceding claims, **characterized in that** the reception zone (100) further includes a transfer material (502).

6. The lateral flow device according to one of the preceding claims, **characterized in that** at least one of the draining materials (211, 212) includes a nanoporous or mesoporous material.

7. The lateral flow device according to one of claims 2 to 6, **characterized in that** the capture zone (400) is located upstream of the conjugation material (501a, 501b) in the direction of drainage (F1, F2) of the liquid in the draining material (211, 211a, 211b, 212a, 212b).

8. The lateral flow device according to one of claims 2 to 7, **characterized in that** at least one of the conjugation materials (501a, 501b) includes conjugation particles capable of binding to an analyte which is certain to be found in the liquid.

9. The lateral flow device according to one of the preceding claims, **characterized in that** the first absorbent material (111), the draining material (211, 212) and the second absorbent material (311, 312) are disposed on a support material (800).

10. The lateral flow device according to one of the preceding claims, **characterized in that** it further includes a cover material (700) capable of covering at least one portion of the reception zone, the migration area and/or the reservoir zone.

11. The lateral flow device according to one of claims 1 to 10, **characterized in that** the capture element (411) is held against the draining material (211).

12. A system for sampling analytes of interest in a liquid, **characterized in that** it includes an enclosure (1000) enclosing a lateral flow device (10) according to one of the preceding claims 1 to 11, including a single migration zone (201), said enclosure including:
- a first opening (1210) disposed facing the reception zone (100) of said device,
- a first window (1230) disposed facing the detection zone (600) in order to allow reading a detection signal (601, 602),

13. The system for sampling analytes of interest in a liquid, **characterized in that** it includes an enclosure (2000) enclosing a lateral flow device (20) according to one of claims 1 to 11, including two migration zones (201, 202), said enclosure including:
- a first opening (2210) disposed facing the reception zone (100) of said device,
- a first window (2220) disposed facing the first detection zone (600a) in order to allow reading a first detection signal (601, 602),
- a second window (2230) disposed facing the second detection zone (600b) in order to allow reading a second detection signal (603).

14. The system for sampling analytes of interest in a liquid according to claim 12 or 13, **characterized in that** the enclosure (1000, 2000) further includes a second opening (1220) disposed facing the capture zone (400) of the device, so as to be able to deposit a capture element (411) on said capture zone and/or remove it therefrom.

15. The system for sampling analytes of interest in a liquid according to one of claims 12 to 14, **characterized in that** the device (10, 20) further comprises at least one capture element (411) held on the capture zone (400), said at least one capture element being capable of capturing an analyte of interest present in the liquid.

16. The system for sampling analytes of interest in a liquid according to one of claims 12 to 15, **characterized in that** the capture element (411) is fastened to a support detachable from the enclosure.

17. The system for sampling analytes of interest in a liquid according to claim 16, **characterized in that** the detachable support of the enclosure is one of the following elements:
- a plate (1400) capable of being fastened at least temporarily on the edges of the second opening (1220),
- a breakable portion (2000a) of the enclosure (2000),
- a plug (3410) capable of being fastened both on a capsule (3400) and on the second opening (3220).

18. A method for sampling at least one analyte of interest in a sample, including the following steps:
- depositing said sample on the reception zone of a device according to any one of claims 1 to 11, and
- waiting until the liquid has migrated to the reservoir zone, **characterized in that** in the case of presence of the analyte of interest in the sample, proven by the detection means, said analyte is sampled from the capture element (411).

19. The sampling method according to claim 18, **characterized in that** the device is comprised in a sampling system according to any one of claims 12 to 17, and that at the end of the liquid migration step, the capture element (411) is separated from the rest of the device.

20. A method for assaying at least one analyte of interest in a liquid sample, including a step of sampling said analyte by a method according to one of claims 18 or 19, followed by a step of assaying said analyte present in the capture element.
